# EUROPEAN PATENT SPECIFICATION

(11) **EP 0 788 386 B1**
(45) Date of publication and mention of the grant of the patent: **13.01.1999**
(21) Application number: 95934724.6
(22) Date of filing: 20.10.1995
(51) Int. Cl.: A61M 5/30, C12M 3/00

(54) **NEEDLELESS SYRINGE**
NADELLOSE SPRITZE
SERINGUE SANS AIGUILLE

(30) Priority: 24.10.1994 GB 9421394
(43) Date of publication of application: 13.08.1997
(73) Proprietor: PowderJect Research Limited, Oxford OX4 4GA (GB)
(72) Inventor: BELLHOUSE, Brian, John, Oxfordshire OX5 2SQ (GB); DRAYSON, Paul, Rudd, Oxfordshire OX8 5RN (GB); GREENFORD, John, Christopher, Oxfordshire OX14 1DF (GB); SARPHIE, David, Francis, Oxfordshire OX8 5SH (GB)
(74) Representative: Jackson, Peter Arthur
(86) International application number: GB9502498
(87) International publication number: WO9612513

(56) References cited:
- EP-A- 0 387 222
- WO-A-95/19799
- US-A- 3 853 125
- US-A- 3 906 950
- US-A- 3 949 751
- US-A- 3 998 226

## Description

In our earlier international patent application No. WO 94/24263, which was unpublished at the priority date hereof, we disclose a non-invasive drug delivery system involving the use of a needleless syringe which fires light drug-containing particles in controlled doses into the intact skin or delivers genetic material into living cells. The syringe described in the earlier application is constructed as an elongate tubular nozzle, a rupturable membrane initially closing the passage through the nozzle adjacent to the upstream end of the nozzle, particles of a therapeutic agent, particularly a powdered therapeutic agent, located adjacent to the membrane, and energizing means for applying to the upstream side of the membrane a gaseous pressure sufficient to burst the membrane and produce through the nozzle a supersonic gas flow in which the particles are entrained.

By appropriate selection of the geometry and Mach number for the nozzle, which preferably has a convergent upstream portion, leading through a throat to a cylindrical or, preferably, divergent downstream portion, it has been possible to provide a pseudo-steady state, supersonic two phase flow through the nozzle, in which the particles move with a velocity close to that of the propelling gas in which they are entrained. Consequently, a large proportion of the particles reach the target under quasi-steady flow conditions and only a small proportion are delivered in transient flow and carried on the contact surface. This leads to considerable benefit both in control and in increased skin or other target penetration and is surprising in such a transient phenomenon.

In the earlier application it was proposed that the particles be contained within a capsule consisting of a pair of the rupturable membranes arranged face to face transversely to the axis of the nozzle and sealed together around their edges by means of an intervening ring provided with sealing means for sealing the periphery of the capsule to a tubular body of the syringe. A capsule of this construction is quite complex for a disposable part and may provide an uncertain dose of the therapeutic agent if a proportion of the particles become entrapped behind the edges of the downstream membrane upon delivery.

US-A-3853125 discloses a needleless syringe comprising a nozzle, an open ended chamber arranged, in use, to contain and enclose a container containing a therapeutic agent, and energizing means for applying a gaseous pressure sufficient to force the agent out through the downstream end of the container and the open downstream end of the chamber and thus to create through the nozzle a gas flow in which the agent is entrained. According to the present invention, such a syringe is characterised in that the nozzle is an elongate nozzle, the chamber is at the upstream end of the elongate nozzle in axial alignment therewith, the chamber is arranged, in use, to contain and enclose a soft-walled capsule as a container containing particles of a therapeutic agent, a means for piercing the upstream end of a capsule in the chamber is arranged at the upstream end of the chamber, and in that a supersonic gas flow is created through the nozzle.

The capsule for use in the new syringe may have a gelatine wall and be substantially cylindrical with domed ends, such as are commonly used in, for example, inhalers used by asthmatics. By using tried and tested technology for creating and filling the capsules, the development and launching of the syringe can be carried out speedily and at low cost.

The capsule chamber for intimately enclosing the capsule may be formed within two separable wall parts which are divided transversely to the axis of the nozzle. In use the two parts will be separated to insert a capsule and then drawn axially together around the capsule. The two wall parts of the chamber may be held together by entrapment between two parts of the syringe body, which are, for example, interconnected by a screw-threaded, bayonet or other releasable connection.

The means for piercing the upstream end of the capsule may be a tubular or other cutter projecting into the capsule chamber from the upstream end of the chamber wall, and being arranged to pierce the upstream end of the capsule as the two parts of the chamber wall are drawn together. Alternatively the upstream end of the capsule may be pierced by a skewer which extends into the chamber through the opening at its upstream end.

In order to enable the particles to be readily forced out through the downstream end of the capsule and through the open downstream end of the capsule chamber and hence into the nozzle, the downstream end of the capsule may be pierced similarly to the upstream end. Alternatively, the downstream end of the capsule may be moulded or otherwise formed with a weakened portion, such as in a cruciform shape, so that the downstream end of the capsule readily ruptures when the necessary gas pressure is applied to initiate the flow through the capsule and along the nozzle.

In order to increase the pressure build-up prior to the supersonic flow, and hence to increase the supersonic velocity, the interconnection between the capsule chamber and the nozzle may be closed initially by a rupturable membrane of, for example, Mylar,(Trade Mark), extending across the axis. In a similar position, a fine mesh may be provided in order to retain any parts of the capsule wall which might otherwise be entrained in the gas flow through the nozzle.

The energizing means may take any of the forms referred to in our earlier application, for example a pressure chamber upstream of the capsule chamber and means for the controlled build-up of gaseous pressure in the pressure chamber, or a pressure chamber upstream of the capsule chamber containing a reservoir of compressed gas, together with means for releasing the pressure from the reservoir for drug delivery.

In other respects, for example in the use of a spacer/silencer at the downstream end of the nozzle, reference is made to the earlier application.

An example of a syringe constructed in accordance with the present invention is illustrated in the accompanying drawings, in which:
Fig. 1 is an axial section;
Fig. 2 is an elevation;
Fig. 3 is a side elevation of a capsule; and,
Fig. 4 corresponds to part of Fig. 1 but of a modified syringe.

The illustrated syringe has a barrel portion formed by rigidly interconnected parts 3 and 4 and a tubular nozzle 5, which has a convergent/divergent passage 5A, and to which the barrel part 4 is interconnected by screw-threads 6. The lower end of the nozzle 5 is provided with a shroud 7 and silencer 8. The barrel part 4 has a passageway 9 interconnecting the interior of the barrel part 3 with a compartment 10 via a ring of ducts 11. The barrel part 3 is arranged to receive through its open upper end a bulb 12 containing pressurized gas and having a neck 13 which is insertable downwards into the passage 9, to which it is sealed by a O-ring 14. The outlet from the bulb 12 through the neck 13 is closed by a spring-loaded ball valve 15. A spigot 16 which is fixed in the barrel part 4 and extends into the neck 13 of the bulb, is arranged to open the ball valve against spring action when the bulb is pushed further than the illustrated position down into the barrel part 3, e.g. by the thumb of a person's hand holding the barrel in its palm.

Mounted between the barrel part 4 and the nozzle 5 is a capsule holder formed by two hollow parts 17 and 18. When brought together as illustrated, these parts define an internal capsule chamber 19 of substantially cylindrical shape with domed ends, the chamber being arranged to contain a soft-walled, powdered drug containing capsule 19A of complementary shape. The parts 17 and 18 are held together between the nozzle 5 and an inwardly projecting rib 20 on the upper barrel 4 when the barrel part 4 is screwed on to the nozzle. The remote ends of the parts 17 and 18 are both provided with fixed tubular cutters 21, 21A which are arranged to pierce the ends of the capsule when, with the capsule between the parts 17 and 18, the parts 17 and 18 are drawn together by screwing up of the barrel part 4 on to the nozzle 5. In this assembled configuration, depression of the end of the bulb down into the barrel part 3 causes the spigot 16 to open the valve 15 and to release the gas pressure within the bulb, which then flows through ducts 11, compartment 10, and into the upper end of the capsule chamber 19. When sufficient pressure has built up, a supersonic flow is created through the interior passage in the nozzle 5, with the particles being flushed out of the capsule and entrained in the gas flow, and hence carried out through the shroud into a patient's skin.

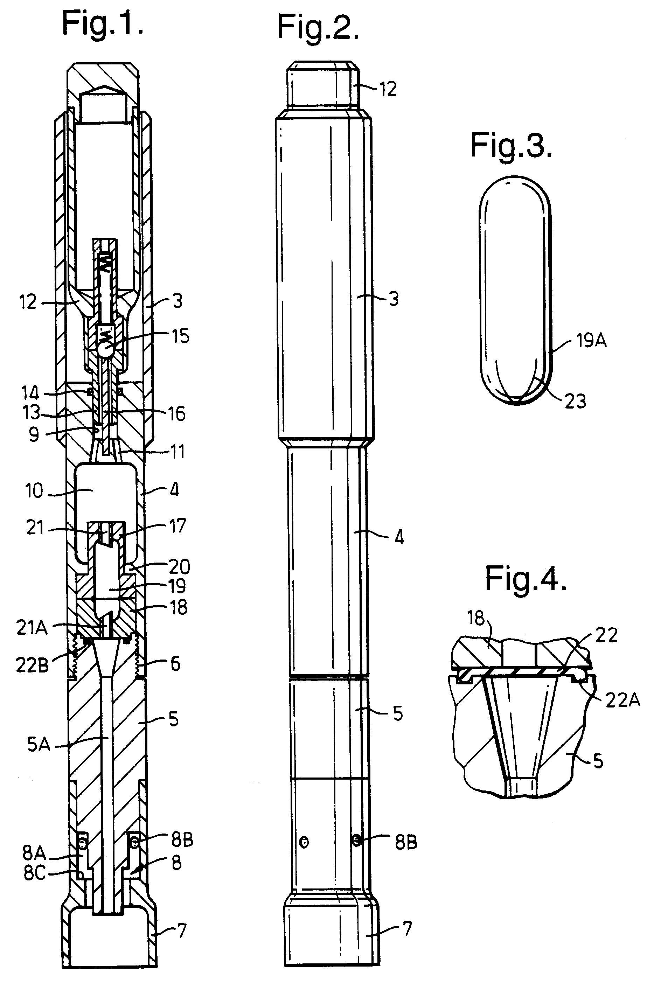

A Mylar(Trade Mark) membrane 22, rupturable to release the gas flow, and/or a screen, may be trapped between the bottom of the chamber part 18 and the upper end of the nozzle 5 as shown in Fig. 4, for the previously explained reasons. The membrane 22 is formed at its edges with a lip 22A, which is received in an annular grove in the top face of the nozzle 5, to seal the parts 5 and 18 together. The lip 22A thus replaces the O-ring 22B shown in Fig. 1.

After discharge of the syringe, the barrel part 4 will be unscrewed from the nozzle 5 to enable removal and disposal of the remnants of the capsule, and also of the Mylar membrane if used. The bulb 12 will also be withdrawn for disposal. In most cases the remaining parts of the syringe may be reused with new disposable parts.

Instead of the lower cutter 21, the lower end of the capsule 19A may be weakened, e.g. by cruciform lines of weakness 23, as shown in Fig. 3. The diaphragm 22 should not then be needed.

The shroud and silencer 7,8 are similar to those described in the earlier application to the extent that the shroud is a tubular part extending beyond the end of the nozzle 5, and the silencer includes an annular passage 8A between an upper portion of the shroud 7 and a lower portion of the nozzle 5, the passage leading from within the shroud 7 to a ring of vents 8B opening out through the upper part of the shroud to the atmosphere. The interior of the passageway is irregular in the sense that both the inner wall of the shroud and the outer wall of the nozzle are stepped as shown at 8C thereby providing surfaces for the flow resulting from reflection of the shockwave at the patient's skin, to make multiple reflections, and thus dissipating the energy and noise. There may be a plurality of the steps 8C at axially spaced positions along the shroud and nozzle, with at least some adjacent pairs facing one another diagonally across the passage 8A, in a similar way to that in which the steps 8C face one another.

Alternatively, instead of being provided with the steps 8C, the passage may be filled with a helical vane, which causes multiple reflections from the adjacent axially facing turns of the vane as the gas flow passes generally helically along the passage. Such helical vane may be formed by moulding a helical flight on the outside of the nozzle and a complementary helical flight on the inside of the shroud, the two being brought into angular alignment when the shroud is fitted to the nozzle.

Typically, the gas provided in the chamber 14 may be helium at a pressure of the order of 40 to 80 bar. The nozzle may be of convergent/divergent, or convergent/cylindrical form with a length of between 50 and 100, preferably 60mm, and a throat diameter of between 1 and 10, preferably between 1.5 and 5mm. With appropriate gas pressure, particles having a diameter of 10-40µm will be accelerated through the nozzle to velocities of between Mach 1 and 3.

## Claims

1. A needleless syringe comprising a nozzle (5), an open ended chamber (19) arranged, in use, to contain and enclose a container (19A) containing a therapeutic agent, and energizing means (12) for applying a gaseous pressure sufficient to force the agent out through the downstream end of the container and the open downstream end of the chamber (19) and thus to create through the nozzle (5) a gas flow in which the agent is entrained; characterised in that the nozzle (5) is an elongate nozzle, the chamber (19) is at the upstream end of the elongate nozzle (5) in an axial alignment therewith, the chamber (19) is arranged, in use, to contain and enclose a soft-walled capsule (19A) as a container containing particles of a therapeutic agent, a means (21) for piercing the upstream end of a capsule (19A) in the chamber is arranged at the upstream end of the chamber (19), and in that a supersonic gas flow is created through the nozzle (5).

2. A syringe according to claim 1, containing a capsule (19A) have a gelatine wall and being substantially cylindrical with domed ends.

3. A syringe according to claim 1 or claim 2, in which the capsule chamber (19) is formed within two separable wall parts (17, 18) which are divided transversely to the axis of the nozzle.

4. A syringe according to claim 3, in which the two wall parts (17, 18) of the chamber (19) are held together by entrapment between two parts (4,5) of the syringe which are interconnected by a releasable connection (6).

5. A syringe according to any one of the preceding claims, in which the means for piercing the upstream end of the capsule is a cutter (21) projecting into the capsule chamber (19) from the upstream end of the chamber.

6. A syringe according to claim 5 when dependent upon claim 3 or claim 4, in which the two wall parts (17,18) are arranged to be drawn axially together around the capsule, the cutter (21) being arranged to pierce the upstream end of the capsule as the two wall parts are drawn together.

7. A syringe according to any one of the preceding claims, further comprising means (21A) at the downstream end of the capsule chamber (19) for piercing the downstream end of a capsule (19A) in the chamber.

8. A syringe according to any one of claims 3 to 6 when dependent upon claim 2, in which the capsule (19A) is formed with a weakened portion (23) so that the downstream end of the capsule readily ruptures when necessary gas pressure is applied to initiate the flow through the capsule and along the nozzle (5).

9. A syringe according to any one of claim 1 to 7, in which the interconnection between the capsule chamber (19) and the nozzle (5) is additionally closed by a rupturable membrane extending across the axis of the nozzle.

## Patentansprüche

1. Nadellose Spritze, aufweisend: eine Düse (5), eine Kammer (19) mit offenem Ende, die angeordnet ist, um in Gebrauch einen Behälter (19A) mit einem therapeutischen Wirkstoff zu enthalten und zu umhüllen, und eine Einrichtung (12) zur Versorgung mit Energie, um einen Gasdruck anzulegen, welcher ausreicht, den Wirkstoff durch das abströmseitige Ende des Behälters und das offene abströmseitige Ende der Kammer (19) hindurchzutreiben, und auf diese Weise durch die Düse (5) eine Gasströmung zu erzeugen, in welcher der Wirkstoff mitgerissen wird; dadurch gekennzeichnet, daß die Düse (5) eine langgestreckte Düse ist, die Kammer (19) sich am einströmseitigen Ende der langgestreckten Düse (5) in axialer Ausrichtung mit dieser befindet, die Kammer (19) angeordnet ist, um in Gebrauch eine Kapsel (19A) mit weicher Wandung als Behälter, welcher Partikel eines therapeutischen Wirkstoffs enthält, zu enthalten und einzuhüllen, eine Einrichtung (21) zum Durchstechen des einströmseitigen Endes einer in der Kammer befindlichen Kapsel (19A) am einströmseitigen Ende der Kammer (19) angeordnet ist, und daß eine Überschallgasströmung durch die Düse (5) hindurch erzeugt wird.

2. Spritze nach Anspruch 1, welche enthält: eine Kapsel (19A), welche eine Gelatinewandung besitzt und im wesentlichen zylindrisch mit bauchigen Enden ist.

3. Spritze nach Anspruch 1 oder 2, bei der die Kapselkammer (19) innerhalb zwei trennbarer Wandteile (17,18) ausgebildet ist, welche in Querrichtung zur Achse der Düse geteilt sind.

4. Spritze nach Anspruch 3, bei der die beiden Wandteile (17,18) der Kammer (19) durch Einschluß zwischen zwei Teile (4, 5) der Spritze zusammengehalten werden, welche durch eine lösbare Verbindung (6) miteinander verbunden sind.

5. Spritze nach einem der vorstehenden Ansprüche, bei der die Einrichtung zum Durchstechen des einströmseitigen Endes der Kapsel eine Schneideeinrichtung (21) ist, welche in die Kapselkammer (19) vom einströmseitigen Ende der Kammer hineinragt.

6. Spritze nach Anspruch 5 wenn dieser von Anspruch 3 oder 4 abhängt, bei der die beiden Wandteile (17, 18) so angeordnet sind, daß sie gemeinsam in axialer Richtung um die Kapsel gezogen werden, und die Schneideeinrichtung (21) so angeordnet ist, daß das einströmseitige Ende der Kapsel durchstochen wird, wenn die beiden Wandteile gemeinsam gezogen werden.

7. Spritze nach einem der vorstehenden Ansprüche, weiter aufweisend: eine Einrichtung (21A) am abströmseitigen Ende der Kapselkammer (19), um das abströmseitige Ende einer in der Kammer befindlichen Kapsel (19A) zu durchstechen.

8. Spritze nach einem der Ansprüche 3 bis 6 wenn diese von Anspruch 2 abhängen, bei der die Kapsel (19A) mit einem geschwächten Abschnitt (23) ausgebildet ist, so daß das abströmseitige Ende der Kammer leicht zerreißt, wenn der erforderliche Gasdruck angelegt wird, um die Strömung durch die Kapsel und entlang der Düse (5) auszulösen.

9. Spritze nach einem der Ansprüche 1 bis 7, bei der die Verbindung zwischen der Kapselkammer (19) und der Düse (5) zusätzlich durch eine zerreißbare Membran verschlossen ist, welche sich quer zur Achse der Düse erstreckt.

## Revendications

1. Seringue sans aiguille comprenant une tuyère (5), une chambre à extrémités ouvertes (19) agencée, pendant l'utilisation, pour contenir et entourer un récipient (19A) contenant un agent thérapeutique, et un moyen d'actionnement (12) pour appliquer une pression gazeuse suffisante pour expulser l'agent par l'extrémité aval du récipient et l'extrémité aval ouverte de la chambre (19) et donc pour créer dans la tuyère (5) un écoulement gazeux dans lequel l'agent est entraîné, caractérisée en ce que la tuyère (5) est une tuyère allongée, la chambre (19) est à l'extrémité amont de la tuyère allongée (5) en alignement axial avec celle-ci, la chambre (19) est agencée, pendant l'utilisation, pour contenir et entourer une capsule à paroi molle (19A) en tant que récipient contenant des particules d'un agent thérapeutique, un moyen (21) pour percer l'extrémité amont d'une capsule (19A) dans la chambre est agencé à l'extrémité amont de la chambre (19), et en ce qu'un écoulement gazeux supersonique est créé dans la tuyère (5).

2. Seringue selon la revendication 1, contenant une capsule (19A) ayant une paroi en gélatine et étant sensiblement cylindrique avec des extrémités bombées.

3. Seringue selon la revendication 1 ou la revendication 2, dans laquelle la chambre à capsule (19) est formée à l'intérieur de deux parties de paroi séparables (17, 18) qui sont divisées transversalement à l'axe de la tuyère.

4. Seringue selon la revendication 3, dans laquelle les deux parties de paroi (17, 18) de la chambre (19) sont maintenues ensemble par inclusion entre deux parties (4, 5) de la seringue qui sont reliées entre elles par une liaison réversible (6).

5. Seringue selon l'une quelconque des revendications précédentes, dans laquelle le moyen pour percer l'extrémité amont de la capsule est un couteau (21) qui fait saillie dans la chambre à capsule (19) depuis l'extrémité amont de la chambre.

6. Seringue selon la revendication 5, lorsqu'elle dépend de la revendication 3 ou de la revendication 4, dans laquelle les deux parties de paroi (17, 18) sont agencées pour être réunies axialement autour de la capsule, le couteau (21) étant agencé pour percer l'extrémité amont de la capsule lorsque les deux parties de paroi sont réunies ensemble.

7. Seringue selon l'une quelconque des revendications précédentes, comprenant en outre un moyen (21A) à l'extrémité aval de la chambre à capsule (19) pour percer l'extrémité aval d'une capsule (19A) dans la chambre.

8. Seringue selon l'une quelconque des revendications 3 à 6, lorsqu'elles sont dépendantes de la revendication 2, dans laquelle la capsule (19A) est munie d'une partie affaiblie (23) de sorte que l'extrémité aval de la capsule est crevée aisément lorsqu'une pression gazeuse nécessaire est appliquée pour amorcer l'écoulement dans la capsule et le long de la tuyère (5).

9. Seringue selon l'une quelconque des revendications 1 à 7, dans laquelle l'interconnexion entre la chambre à capsule (19) et la tuyère (5) est fermée en outre par une membrane crevable qui s'étend transversalement à l'axe de la tuyère.
